# FASCICULE DE BREVET EUROPEEN

(11) **EP 3 237 470 B1**
(45) Date de publication et mention de la délivrance du brevet: **15.07.2020**
(21) Numéro de dépôt: 15800882.1
(22) Date de dépôt: 30.10.2015
(51) Int. Cl.: C08F 220/10, A61Q 5/00, A61K 8/00, C08F 222/10, C08L 33/06, C08F 220/06

(54) **POLYMÈRE MULTIPHASIQUE A TITRE D'AGENT ÉPAISSISSANT ET SUSPENSIF**
MEHRPHASIGES POLYMER ALS VERDICKUNGSMITTEL UND SUSPENSIONSMITTEL
MULTIPHASE POLYMER AS A THICKENING AND SUSPENDING AGENT

(30) Priorité: 23.12.2014 FR 1463229
(43) Date de publication de la demande: 01.11.2017
(73) Titulaire: COATEX, 69730 Genay (FR)
(72) Inventeur: CHAMPAGNE, Clémentine, F-69300 Caluire-Et-Cuire (FR); SUAU, Jean-Marc, F-69480 Lucenay (FR); MAGNY, Benoît, F-69270 Cailloux Sur Fontaines (FR); BONY, Delphine, F-69650 Quincieux (FR); KENSICHER, Yves, F-69620 Theize (FR)
(74) Mandataire: Balmefrezol, Ludovic Francis Pierre
(86) Numéro de dépôt international: PCT/FR2015/052934
(87) Numéro de publication internationale: WO 2016/102790

(56) Documents cités:
- US-A- 5 362 415
- US-A1- 2010 184 897
- US-A1- 2011 207 856
- US-A1- 2012 231 056
- US-A1- 2013 115 185
- US-A1- 2014 179 580
- US-A1- 2014 179 590

## Description

La présente invention concerne de nouveaux polymères utilisables comme agents modificateurs de rhéologie pour des formulations aqueuses et permettant d'induire à la fois de bonnes propriétés d'épaississement, de clarté et de bonnes performances suspensivantes.

Les agents modificateurs de rhéologie, également appelés agents épaississants ou de viscosité, sont présents dans les compositions nettoyantes, que ce soit dans les compositions de soin ou d'hygiène de la personne, par exemple les compositions cosmétiques, ou dans les compositions d'entretien telles que des produits détergents. Ces agents influencent les propriétés rhéologiques (en particulier la viscosité) et esthétiques (telles que la clarté) de la formulation généralement riche en tensioactifs, ainsi que la capacité à suspendre et à stabiliser des particules au sein de la formulation.

Parmi les agents modificateurs de rhéologie couramment utilisés dans les formulations aqueuses, on peut citer les polymères solubles ou gonflables en milieu alcalin, plus connus sous l'appellation « ASE » (pour « Alkali-Soluble or Swellable Emulsions » en anglais) et les polymères solubles ou gonflables en milieu alcalin et modifiés hydrophobes, plus connus sous l'appellation « HASE » (pour « Hydrophobically modified Alkali-Soluble or Swellable Emulsions » en anglais). Les polymères de type ASE sont des copolymères linéaires ou ramifiés synthétisés à partir d'acide (méth)acrylique et d'acrylates d'alkyle. Les polymères HASE sont des copolymères linéaires ou ramifiés synthétisés à partir d'acide (méth)acrylique, d'acrylates d'alkyle et d'au moins un monomère associatif.

Ces agents modificateurs de rhéologie permettent d'accéder à de bonnes propriétés suspensivantes, de viscosité et de clarté dans des formulations riches en tensioactifs présentant des valeurs de pH proches de la neutralité. Malheureusement, ils ne permettent pas de combiner, dans des conditions de pH acide, de bonnes propriétés suspensivantes, de viscosité et de clarté.

Or, il est souhaitable de pouvoir formuler des compositions à base de tensioactifs dans une gamme de pH correspondant à celui de la peau, c'est-à-dire à des valeurs de pH comprises entre 4 et 6 suivant que le produit cosmétique est destiné à une application sur le corps, le visage ou encore les muqueuses.

De plus, les conservateurs classiquement utilisés tels que les donneurs formaldéhydes, les composés halogénés et les composés parabènes sont soit interdits d'utilisation, soit soupçonnés d'avoir des effets néfastes sur la santé. Pour les remplacer, les acides organiques (par exemple l'acide sorbique, citrique et benzoïque), tels qu'utilisés dans l'industrie alimentaire, se présentent comme une alternative intéressante. Toutefois, l'activité antimicrobienne des acides organiques est liée aux espèces associées ou protonées de la molécule acide. Lorsque le pH de la formulation contenant l'acide organique augmente, des sels d'acides sont formés par dissociation du proton. Or, la forme dissociée des acides organiques n'a pas d'activité antimicrobienne lorsqu'elle est utilisée seule, ce qui ne permet pas l'utilisation de ces composés acides organiques dans des formulations présentant des valeurs de pH supérieures à 6.

Il a, par ailleurs, été suggéré dans la littérature que la formulation de produits dans une gamme de pH acide permet de réduire la quantité de conservateurs requis dans le produit en améliorant son efficacité, de stabiliser et d'accroître l'efficacité d'ingrédients actifs cosmétiques, ce qui est bénéfique dans la réparation et l'entretien de la barrière cutanée et renforce la flore cutanée (Cosmetics & Toiletries®, vol. 123, N° 12 de décembre 2008, « Formulating at pH 4-5 : How Lower pH Benefits the Skin and Formulations »).

Le document WO 2012/006402 décrit des polymères de type cœur-écorce comprenant un polymère cœur linéaire à base d'unités acryliques et un polymère écorce réticulé à base d'unités acryliques. Ces polymères cœurs-écorces sont proposés pour conduire à de bonnes propriétés esthétiques, de rhéologie et de clarté dans des compositions aqueuses comprenant des tensioactifs dans des conditions de pH acide. Toutefois, il apparaît difficile d'obtenir une composition incorporant les polymères cœurs-écorces décrits et combinant à la fois de bonnes performances suspensivantes, une viscosité et une clarté élevées dans des conditions de pH acide.

La présente invention vise à proposer de nouveaux agents modificateurs de rhéologie, présentant à la fois de bonnes propriétés en termes d'effet épaississant (viscosité) et permettant de conduire à des formulations présentant de bonnes performances suspensivantes et une clarté élevée (phase continue limpide), même dans des conditions de pH acide (pH ≤ 6).

Les inventeurs ont découvert qu'il est possible d'accéder à une formulation répondant à l'ensemble de ces critères (viscosité, performances suspensivantes et clarté) en mettant en œuvre à titre d'agent modificateur de rhéologie un polymère multiphasique spécifique. Plus particulièrement, la présente invention concerne, selon un premier de ses aspects, un polymère multiphasique comprenant de 45 % à 95 % en poids d'un premier polymère, noté P1, et de 5 % à 55 % en poids d'un second polymère, noté P2, les polymères P1 et P2 étant de compositions distinctes.
(1) Ledit polymère P1 étant obtenu par polymérisation à partir d'un mélange de monomères, distincts les uns des autres, comprenant :
   - au moins un monomère (a) anionique présentant une fonction vinylique polymérisable,
   - au moins un monomère (b) hydrophobe non ionique présentant une fonction vinylique polymérisable,
   - au moins un monomère (c) réticulant,
   - au moins un monomère (d) associatif présentant une fonction vinylique polymérisable et une chaîne hydrocarbonée hydrophobe et
   - éventuellement au moins un monomère (e) non ionique additionnel, distinct du monomère (b).
(2) Ledit polymère P2 étant obtenu par polymérisation à partir d'un mélange de monomères, distincts les uns des autres, comprenant :
   - au moins un monomère (a') anionique présentant une fonction vinylique polymérisable,
   - au moins un monomère (b') hydrophobe non ionique présentant une fonction vinylique polymérisable,
   - au moins un monomère (c') réticulant,
   - éventuellement au moins un monomère (d') associatif présentant une fonction vinylique polymérisable et une chaîne hydrocarbonée hydrophobe et
   - éventuellement au moins un monomère (e') non ionique additionnel, distinct du monomère (b').

De manière avantageuse, comme illustré dans les exemples qui suivent, les polymères multiphasiques selon l'invention procurent à la formulation dans laquelle ils sont mis en œuvre de bonnes propriétés suspensivantes, d'épaississement et de clarté, même lorsqu'ils sont formulés à pH acide.

Un polymère multiphasique présentant les compositions spécifiques P1 et P2 précisées ci-dessus est désigné plus simplement dans la suite du texte sous l'appellation « polymère selon l'invention ».

Par l'expression «polymère multiphasique », on entend désigner au sens de l'invention une particule multiphasique de polymère, autrement dit une particule de polymère présentant une composition non homogène, préparée par un procédé de polymérisation séquentielle en au moins deux étapes à partir d'au moins deux compositions de monomères distinctes.

Les particules multiphasiques selon l'invention peuvent être notamment structurées en cœur/écorce (ou « core/shell » en anglais), le premier polymère formant le « cœur » et le second polymère formant l'« écorce ». Cette appellation « cœur/écorce » ne doit toutefois pas être interprétée comme désignant une particule dans laquelle la partie « cœur » serait totalement revêtue ou encapsulée par une partie « écorce », mais comme désignant une particule à morphologie contrôlée présentant deux phases distinctes.

L'expression «polymère P1 » peut s'entendre d'un unique polymère P1 tel que défini ci-dessus ou de plusieurs polymères P1 obtenus par polymérisation séquentielle.

De même, l'expression « polymère P2 » peut s'entendre d'un unique polymère P2 tel que défini ci-dessus ou de plusieurs polymères P2 obtenus par polymérisation séquentielle.

Par « propriétés suspensivantes » ou « pouvoir suspensif », on entend désigner l'aptitude de la composition à maintenir en suspension des particules dans sa phase continue, en particulier de manière stable dans le temps, par exemple lors du stockage de la composition.

Par « particules » à suspendre, on entend des corps solides, pleins ou creux, liquides non miscibles avec la formulation ou encapsulés ou gazeux qui peuvent être caractérisés par des formes, des textures, des structures, des compositions, des couleurs et des propriétés finales différentes. A titre indicatif, on peut citer les particules exfoliantes (par exemple les particules de polyéthylène, les coquilles de fruits pillés, les pierres ponces), les particules nourrissantes (par exemple les sphères de collagène), les particules nacrantes (par exemple le mica titane, les glycols distéarates) et les particules esthétiques (par exemple les bulles d'air, les paillettes, les pigments éventuellement colorés). Pour ce qui est de la suspension de bulles d'air dans la composition, les particules peuvent notamment avoir une taille de 1, 2 ou 3 mm.

Les performances suspensivantes peuvent être évaluées par observation de la stabilité de la suspension de particules normalisées dans une composition stockée à l'étuve à 45°C, comme décrit dans les exemples qui suivent.

La « clarté » ou « limpidité » de la composition peut être évaluée par mesure de la transmittance de la composition. Une méthode de détermination de la transmittance est décrite dans les exemples qui suivent. Elle est exprimée en pourcentage. Une composition est considérée comme claire ou limpide si elle présente une transmittance, pour une longueur d'onde de 500 nm, d'au moins 60 %, de préférence d'au moins 70 % et plus préférentiellement encore d'au moins 80 %.

D'autres caractéristiques, avantages et modes d'application du polymère multiphasique selon l'invention ressortiront mieux à la lecture de la description et des exemples qui vont suivre, donnés à titre illustratif et non limitatif.

Dans la suite du texte, les expressions « compris entre ... et ... », « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

### Polymère Multiphasique

Comme indiqué précédemment, le polymère multiphasique selon l'invention comprend, en particulier est formé, de 45 % à 95 % en poids d'un premier polymère P1 et de 5 % à 55 % en poids d'un second polymère P2, les compositions des polymères P1 et P2 étant différentes.
(1) Ledit polymère P1 étant obtenu par polymérisation à partir d'un mélange de monomères, distincts les uns des autres, comprenant :
   - au moins un monomère (a) anionique présentant une fonction vinylique polymérisable,
   - au moins un monomère (b) hydrophobe non ionique présentant une fonction vinylique polymérisable,
   - au moins un monomère (c) réticulant,
   - au moins un monomère (d) associatif présentant une fonction vinylique polymérisable et une chaîne hydrocarbonée hydrophobe et
   - éventuellement au moins un monomère (e) non ionique additionnel, distinct du monomère (b).
(2) Ledit polymère P2 étant obtenu par polymérisation à partir d'un mélange de monomères, distincts les uns des autres, comprenant :
   - au moins un monomère (a') anionique présentant une fonction vinylique polymérisable,
   - au moins un monomère (b') hydrophobe non ionique présentant une fonction vinylique polymérisable,
   - au moins un monomère (c') réticulant,
   - éventuellement au moins un monomère (d') associatif présentant une fonction vinylique polymérisable et une chaîne hydrocarbonée hydrophobe et
   - éventuellement au moins un monomère (e') non ionique additionnel, distinct du monomère (b').

Dans la suite du texte, les proportions en monomères entrant dans la composition du polymère P1 (respectivement du polymère P2) sont exprimées en pourcentage en poids par rapport au poids total de monomères mis en œuvre pour former le polymère P1 (respectivement le polymère P2).

Selon un mode de réalisation particulier, le polymère P1 ne comprend pas d'unité monomérique autre que les monomères (a), (b), (c) et (d) (à l'exception de la présence optionnelle de fragments d'agents de transfert ou d'initiateurs de polymérisation).

Selon un mode de réalisation particulier, le polymère P2 ne comprend pas d'unité monomérique autre que les monomères (a'), (b'), (c') et (d') (à l'exception de la présence optionnelle de fragments d'agents de transfert ou d'initiateurs de polymérisation).

Autrement dit, selon une variante de réalisation, la somme des teneurs en monomères (a), (b), (c) et (d) de la composition du polymère P1 (respectivement (a'), (b'), (c') et éventuellement (d') de la composition du polymère P2) est égale à 100 %.

Selon une autre variante de réalisation, le polymère P1 et/ou le polymère P2 peu(ven)t comprendre, en outre, une ou plusieurs unité(s) monomérique(s) additionnelle(s) distincte(s) des monomères (a), (b), (c) et (d) (respectivement (a'), (b'), (c') et (d')).

En particulier, la composition du polymère P1 (respectivement du polymère P2) peut comprendre, en outre, un ou plusieurs monomère(s) additionnel(s) non ionique(s) (e) (respectivement (e')) comme détaillé plus précisément dans la suite du texte.

Par ailleurs, il est entendu que les monomères (a) et (a') (respectivement (b) et (b'), respectivement (c) et (c'), respectivement (d) et (d'), respectivement (e) et (e')) entrant dans la composition du polymère P1 et du polymère P2 peuvent être de même nature dans le polymère P1 et dans le polymère P2 ou de natures différentes.

Les monomères (a), (b), (c), (d) et (e) de la composition du polymère P1 sont différents, c'est-à-dire que les monomères (a), (b), (c), (d) et (e) sont distincts les uns des autres. En particulier, le ou lesdits monomère(s) (b) est(sont) différent(s) du ou desdits monomère(s) (d). En particulier, le ou lesdits monomère(s) (a) est(sont) différent(s) du ou desdits monomère(s) (d). Il en va de même pour les monomères (a'), (b'), (c'), (d') et (e') de la composition du polymère P2. Ainsi, en particulier, le ou lesdits monomère(s) (b') est(sont) différent(s) du ou desdits monomère(s) (d'). En particulier, le ou lesdits monomère(s) (a') est(sont) différent(s) du ou desdits monomère(s) (d').

Selon un mode de réalisation particulier, le rapport pondéral polymère P1/polymère P2 du polymère multiphasique selon l'invention est compris entre 45/55 et 95/5, en particulier entre 60/40 et 95/5.

### Monomère anionique présentant une fonction vinylique polymérisable

Selon un mode de réalisation particulier, les monomères anioniques (a) et (a') présentant une fonction vinylique polymérisable, appelés plus simplement dans la suite du texte « monomères anioniques », comprennent au moins un groupement carboxylique.

En particulier, les monomères anioniques peuvent être choisis parmi l'acide acrylique, l'acide méthacrylique, l'acide maléique, l'acide itaconique, l'acide crotonique et leurs mélanges, et/ou les sels de ces acides.

Selon un mode de réalisation particulier, les monomères anioniques peuvent être choisis parmi les monomères d'acide acrylique et/ou d'acide méthacrylique et/ou l'un de leurs sels.

De préférence, le monomère anionique (a) du polymère P1 et (a') du polymère P2 du polymère multiphasique selon l'invention est l'acide méthacrylique (AMA).

Le ou lesdits monomère(s) anionique(s) (a) peu(ven)t représenter de 20 % à 53 % en poids, en particulier de 25 % à 48 % en poids et plus particulièrement de 30 % à 43 % en poids, par rapport au poids total de monomères formant le polymère P1.

Le ou lesdits monomère(s) anionique(s) (a') peu(ven)t représenter de 10 % à 53 % en poids, en particulier de 15 % à 48 % en poids et plus particulièrement de 20 % à 43 % en poids, par rapport au poids total de monomères formant le polymère P2.

Le ou lesdits monomère(s) anionique(s) (a) et (a') peu(ven)t représenter de 14,5 % à 53 % en poids, en particulier de 19,5 % à 48 % en poids et plus particulièrement de 24,5 % à 43 % en poids, du poids total de monomères formant le polymère multiphasique de l'invention.

### Monomère hydrophobe non ionique présentant une fonction vinylique polymérisable

Les monomères hydrophobes non ioniques (b) et (b') présentant une fonction vinylique polymérisable, appelés plus simplement dans la suite du texte « monomères hydrophobes non ioniques », sont des monomères ne présentant ni charge positive ni charge négative en solution aqueuse.

Ils peuvent être choisis parmi les esters, les amides ou les nitriles des acides acryliques ou méthacryliques ou parmi l'acrylonitrile, le styrène, le méthylstyrène, le diisobutylène, la vinylpyrrolidone ou la vinylcaprolactame.

Tout particulièrement, les monomères hydrophobes non ioniques peuvent être choisis parmi les acrylates d'alkyle en C₁-C₈ ou les méthacrylates d'alkyle en C₁-C₈ tels que l'acrylate de méthyle, l'acrylate d'éthyle, l'acrylate de butyle, l'acrylate de 2-éthyle-hexyle, le méthacrylate de méthyle, le méthacrylate d'éthyle, le méthacrylate de butyle et leurs mélanges.

Selon un mode de réalisation particulier, les monomères hydrophobes non ioniques peuvent être choisis parmi l'acrylate d'éthyle, l'acrylate de butyle, le méthacrylate d'éthyle et leurs mélanges.

En particulier, le monomère hydrophobe non ionique (b) du polymère P1 et (b') du polymère P2 du polymère multiphasique selon l'invention peut être l'acrylate d'éthyle (AE).

Le ou lesdits monomère(s) hydrophobe(s) non ionique(s) (b) peu(ven)t représenter de 40 % à 75 % en poids, en particulier de 45 % à 70 % en poids et plus particulièrement de 50 % à 65 % en poids, par rapport au poids total de monomères formant le polymère P1.

Le ou lesdits monomère(s) hydrophobe(s) non ionique(s) (b') peu(ven)t représenter de 50 % à 85 % en poids, en particulier de 55 % à 80 % en poids et plus particulièrement de 60 % à 75 % en poids, par rapport au poids total de monomères formant le polymère P2. Le ou lesdits monomère(s) hydrophobe(s) non ionique(s) (b) et (b') peu(ven)t représenter de 40 % à 83 % en poids, en particulier de 45 % à 78 % en poids et plus particulièrement de 50 % à 73 % en poids du poids total de monomères formant le polymère multiphasique de l'invention.

Selon un mode de réalisation particulièrement préféré, la proportion massique de monomères (b') dans le polymère P2 (teneur massique en monomères (b') par rapport au poids total de monomères formant le polymère P2) est supérieure à celle dans le polymère P1 (teneur massique en monomères (b) par rapport au poids total de monomères formant le polymère P1).

Le ou lesdits monomère(s) anionique(s) (a) et (a') et le ou lesdits monomère(s) hydrophobe(s) non ionique(s) (b) et (b') peu(ven)t représenter plus de 83 % en poids, en particulier entre 83 % à 99,3 % en poids de la composition globale du polymère multiphasique de l'invention.

Par « composition globale », on entend le poids total des monomères mis en œuvre pour la synthèse du polymère multiphasique.

De préférence, le rapport pondéral monomères hydrophobes non ioniques (b')/monomères anioniques (a') de la composition du polymère P2 est compris entre 60/40 et 85/15, en particulier entre 65/35 et 80/20.

Selon un mode de réalisation particulier, le rapport pondéral monomères hydrophobes non ioniques (b)/monomères anioniques (a) de la composition du polymère P1 est compris entre 53/47 et 70/30, en particulier entre 55/45 et 68/32.

Selon un mode de réalisation particulier, le polymère multiphasique selon l'invention est tel que :
- les monomères anioniques (a) et (a') des polymères P1 et P2 sont choisis parmi l'acide acrylique et/ou l'acide méthacrylique et/ou l'un de leurs sels, en particulier le monomère (a) et le monomère (a') sont l'acide méthacrylique et
- les monomères hydrophobes non ioniques (b) et (b') des polymères P1 et P2 sont choisis parmi l'acrylate d'éthyle, l'acrylate de butyle, le méthacrylate d'éthyle ou leurs mélanges, en particulier le monomère (b) et le monomère (b') sont l'acrylate d'éthyle.

### Monomère réticulant

Le polymère P1 et le polymère P2 du polymère multiphasique selon l'invention sont partiellement ou totalement réticulés.

Les compositions du polymère P1 et du polymère P2 comprennent ainsi toutes les deux, en outre, un ou plusieurs monomère(s) réticulant(s).

Selon un mode de réalisation particulier, le polymère multiphasique selon l'invention comporte un unique monomère réticulant. Selon un autre mode de réalisation, il comporte deux monomères réticulants différents. Le ou les monomère(s) réticulant(s) est(sont) utilisé(s) pour générer un polymère sous forme de réseau tridimensionnel.

Selon la présente invention, on utilise en tant que monomère réticulant, un monomère qui est un composé polyinsaturé. Ce composé peut comporter deux, trois ou plusieurs insaturations éthylèniques.

Le monomère réticulant peut avoir un caractère hydrophile, hydrophobe ou amphiphile.

Des exemples de ces composés incluent les composés di(méth)acrylates comme le di(méth)acrylate de polyalkylène glycol, notamment le di(méth)acrylate de polypropylène glycol, le di(méth)acrylate d'éthylène glycol, le di(méth)acrylate de polyéthylène glycol, le di(méth)acrylate de triéthylène glycol, le di(méth)acrylate de 1,3-butylène glycol, le di(méth)acrylate de 1,6-butylène glycol, le di(méth)acrylate de 1,6-hexanédiol, le di(méth)acrylate de néopentyl glycol, le di(méth)acrylate de 1,9-nonanediol, mais aussi le 2,2'-bis(4-(acryloxy-propyloxyphényl)propane, le 2,2'-bis(4-(acryloxydiéthoxy-phényl)propane et l'acrylate de zinc ; les composés tri(méth)acrylates tels que le tri(méth)acrylate de triméthylolpropane, le tri(méth)acrylate triméthylolethane, le tri(méth)acrylate pentaérythritol et le tri(méth)acrylate de tétramethylolméthane ; les composés tétra(méth)acrylates tels que le tétra(méth)acrylate de ditriméthylolpropane, le tétra(méth)acrylate de tétraméthylolméthane et le tétra(méth)acrylate de pentaérythritol ; les composés hexa(méth)acrylates tels que l'hexa(méth)acrylate de dipentaérythritol ; les composés penta(méth)acrylates tels que le penta(méth)acrylate de dipentaerythritol ; les composés allyls tels que l'allyl (méth)acrylate, le diallylphthalate, l'itaconate de diallyl, le fumarate de diallyl et le maléate de diallyl ; les éthers polyallyls du sucrose ayant de 2 à 8 groupes par molécule, les éthers polyallyls du pentaérythritol tels que le pentaérythritol diallyl éther, le pentaérythritol triallyl éther et le pentaérythritol tetraallyl éther ; les éthers polyallyls du triméthylolpropane tels que l'éther diallyl triméthylolpropane et l'éther triallyl triméthylolpropane. D'autres composés polyinsaturés incluent le divinyl glycol, le divinyl benzène, le divinylcyclohexyl et le méthylènebisacrylamide.

Selon un autre aspect, les monomères réticulants peuvent être préparés par une réaction d'estérification d'un polyol avec un anhydride insaturé tel que l'anhydride maléique ou l'anhydride itaconique ou par une réaction d'addition avec un isocyanate tel que le 3-isopropényl-diméthylbenzène isocyanate.

On peut également utiliser les composés suivants pour obtenir des monomères réticulants : polyhaloalkanols tels que le 1,3-dichloroisopropanol et le 1,3-dibromoisopropanol ; haloepoxyalkanes tels que l'épichlorohydrine, l'épibromohydrine, le 2-méthyl épichlorohydrine et l'épiiodohydrine ; polyglycidyls éthers tels que le 1,4-butanediol diglycidyl éther, glycérine-1,3-diglycidyl éther, éthylène glycol diglycidyl éther, propylène glycol diglycidyl éther, diéthylène glycol diglycidyl éther, néopentyl glycol diglycidyl éther, polypropylène glycol diglycidyl éther, bisphénol A-épichlorohydrine époxy résine et des mélanges.

Selon un mode de réalisation particulier, les monomères réticulants mis en œuvre dans le polymère P1 et dans le polymère P2 sont choisis parmi les réticulants trifonctionnels.

Il peut s'agir en particulier du triacrylate de triméthylolpropane (TMPTA).

La teneur en monomère(s) réticulant(s) (c) dans la composition du polymère P1 peut être plus particulièrement supérieure ou égale à 0,3 % en poids, par rapport au poids total des monomères formant le polymère P1, en particulier supérieure ou égale à 0,4 % en poids et de préférence comprise entre 0,4 % et 5 % en poids.

La teneur en monomère(s) réticulant(s) (c') dans la composition du polymère P2 peut être plus particulièrement supérieure ou égale à 0,2 % en poids, par rapport au poids total des monomères formant le polymère P2, en particulier supérieure ou égale à 0,3 % et notamment comprise entre 0,3 % et 5 % en poids.

Le ou lesdits monomère(s) réticulant(s) (c) et (c') peu(ven)t représenter de 0,2 % à 5 % en poids, en particulier de 0,3 % à 5 % en poids, du poids total de monomères formant le polymère multiphasique de l'invention.

### Monomère associatif

Les monomères associatifs présentant une fonction vinylique polymérisable et une chaîne hydrocarbonée hydrophobe sont de préférence choisis parmi des monomères oxyalkylés. Ils peuvent être plus particulièrement choisis parmi les monomères de formule (I) suivante :

T-[(EO)ₙ(PO)_{n'}(BO)_{n"}]-Z (I)

dans laquelle :
- T représente une extrémité permettant la copolymérisation du monomère associatif,
- [(EO)ₙ(PO)_{n'}(BO)_{n"}] représente une chaîne polyalkoxylée constituée d'unités alkoxylées, réparties en blocs, alternées ou statistiques, choisies parmi les unités éthoxylées EO, les unités propoxylées PO et les unités butoxylées BO,
- n, n', n" représentent, indépendamment les uns des autres, 0 ou un nombre entier variant de 1 à 150 et
- Z représente une chaîne grasse, linéaire ou ramifiée, d'au moins 12 atomes de carbone et comportant éventuellement un ou plusieurs groupe(s) cyclique(s) de 5 à 7 chaînons, saturés, partiellement insaturés ou aromatiques, lesdits groupes pouvant être éventuellement substitués.

De manière équivalente, le monomère associatif peut être représenté selon la formule (II) suivante :

T-A-Z (II)

dans laquelle :
- T représente une extrémité permettant la copolymérisation du monomère associatif,
- A représente une chaîne polymérique constituée de :
   - m motifs d'oxyde d'alkylène de formule -CH₂CHR₁O- avec R₁ représentant un groupement alkyle comprenant de 1 à 4 carbones, par exemple un groupement méthyle ou éthyle, et m variant de 0 à 150,
   - p motifs d'oxyde d'alkylène de formule -CH₂CHR₂O- avec R₂ représentant un groupement alkyle comprenant de 1 à 4 carbones, par exemple un groupement méthyle ou éthyle, et p allant de 0 à 150,
   - n motifs d'oxyde d'éthylène avec n variant de 0 à 150, ou de 10 ou 15 à 150, ou de 10 ou 15 à 100, ou de 15 à 50, ou de 15 à 30,
      dans laquelle m+n+p ≥ 0 et
      dans laquelle les motifs d'oxyde d'alkylène de formule -CH₂CHR₁O-, les motifs d'oxyde d'alkylène de formule -CH₂CHR₂O- et les motifs d'oxyde d'éthylène sont en blocs, alternés ou statistiques et
- Z est tel que défini précédemment.

Par « unités propoxylées PO » et « unités butoxylées BO », on entend des unités éthoxylées porteuses sur l'un ou l'autre de leurs carbones, d'un radical méthyle ou éthyle respectivement. Une unité éthoxylée est une unité -CH₂-CH₂-O.

Par « chaîne grasse », on entend une chaîne hydrocarbonée aliphatique d'un acide gras, linéaire ou ramifiée, comprenant au moins 12 atomes de carbone, ou de 12 à 36 atomes de carbone, ou de 12 à 32 atomes de carbone.

La chaîne Z peut comporter, par exemple, de 2 à 10 groupes cycliques aromatiques.

Selon un mode de réalisation particulier, la chaîne grasse Z peut comporter un ou plusieurs groupement(s) phénol(s) porteur(s) d'un ou plusieurs groupe(s) styryle(s) tel(s) que, par exemple, des groupements distyrylphénols, tristyrylphénols et/ou pentastyrylcumylphénols.

De préférence, la chaîne Z est une chaîne ramifiée comportant 16 atomes de carbone.

L'extrémité T représente plus particulièrement un radical contenant une fonction insaturée polymérisable, appartenant au groupe des esters acryliques, méthacryliques, maléiques, itaconiques ou crotoniques. L'extrémité T peut notamment être choisie parmi les groupements acrylates, méthacrylates, allyliques, vinyliques, méthacryluréthanes et alphas, alpha-diméthyl-m-isopropényls benzyls uréthanes.

Selon un mode de réalisation particulier, le monomère associatif répond à la formule (III) :

CH₂=C(R₁)-COO-[(EO)ₙ(PO)_{n'}(BO)_{n"}]-Z (III)

dans laquelle :
- R₁ représente H ou CH₃ et
- n, n', n" et Z ont la même définition que dans la formule (I) ci-dessus.

De manière équivalente, le monomère associatif répond à la formule (IV) suivante :

CH₂=C(R₁)-COO-A-Z (IV)

dans laquelle :
- R₁ représente H ou CH₃ et
- A et Z ont la même définition que dans la formule (II) ci-dessus.

Selon un mode de réalisation particulier, n' et n" dans la formule (I) ou (III) précitée sont nuls et n varie de préférence de 15 à 150, en particulier de 15 à 50 et notamment de 15 à 30. Autrement dit, A dans les formules (II) et (IV) précitées représente une chaîne polymérique constituée de 15 à 150, en particulier de 15 à 50 et notamment de 15 à 30 motifs d'oxyde d'éthylène.

A titre d'exemple, le monomère associatif peut répondre à la formule (III) dans laquelle n' et n" sont nuls, n vaut 25, R₁ représente CH₃, Z est une chaîne ramifiée comportant 16 atomes de carbone.

Comme indiqué précédemment, le ou lesdits monomère(s) associatif(s) peu(ven)t être présent(s) uniquement dans le polymère P1.

Alternativement, le ou lesdits monomère(s) associatif(s) peu(ven)t être présent(s) à la fois dans le polymère P1 et dans le polymère P2 du polymère multiphasique de l'invention. Le ou lesdits monomère(s) associatif(s) (d), et éventuellement (d'), peu(ven)t représenter au moins 0,5 % en poids de la composition globale du polymère multiphasique de l'invention.

Par « composition globale », on entend le poids total des monomères mis en œuvre pour la synthèse du polymère multiphasique.

En particulier, le ou lesdits monomère(s) associatif(s) (d) peu(ven)t être mis en œuvre à raison d'au moins 0,5 % en poids, en particulier de 0,5 à 12 % en poids, par rapport au poids total de monomères formant le polymère P1.

### Monomères additionnels non ioniques

Les polymères P1 et P2 peuvent comprendre un ou plusieurs monomère(s) additionnel(s) non ionique(s) (e) et/ou (e'). Ces monomères additionnels non ioniques (e) et (e') peuvent être plus particulièrement choisis parmi :
- l'acide 2-acrylamido-2-méthylpropane sulfonique (AMPS),
- les télomères insaturés de l'acide acrylique,
- les monomères de formule (el) : 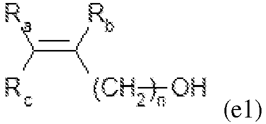 dans laquelle :
   - Ra, Rb et Rc représentent, indépendamment les uns des autres, H ou CH3,
   - n est un entier égal à 1 ou à 2 et
   - les monomères de formule (e2) : 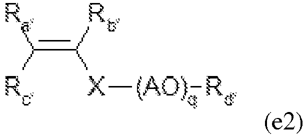 dans laquelle :
      - R_{a'}, R_{b'}, R_{c'} et R_{d'} représentent, indépendamment les uns des autres, H ou CH₃,
      - X représente (C=O) ou (CH₂)ᵣ avec r = 0, 1 ou 2,
      - (AO) représente une chaîne polyalkoxylée constituée d'unités alkoxylées, réparties en blocs, alternées ou statistiques, choisies parmi les unités éthoxylées EO, les unités propoxylées PO et les unités butoxylées BO et
      - q représente 0 ou un nombre entier variant de 1 à 150.

En particulier, les monomères additionnels de formule (el) peuvent être choisis parmi l'alcool allylique (n = 1), l'alcool méthallylique (n = 1) et l'isoprénol (n =2). Avantageusement, le monomère optionnel est l'isoprénol.

Par « télomères insaturés de l'acide acrylique », on entend des oligomères d'acide acrylique ou d'acide acryloxypropionique, de formule (V) : 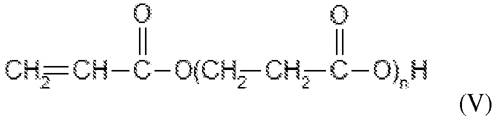 où n est un entier variant de 1 à 10. Ces différents oligomères peuvent être en mélange. Lorsque n = 1, l'oligomère est un dimère d'acide acrylique.

Il est entendu que les différents modes particuliers décrits pour chacun des monomères anioniques, hydrophobes non ioniques, réticulants et associatifs du polymère multiphasique selon l'invention peuvent être combinés.

Le ou lesdits monomère(s) non ionique(s) additionnel(s) (e) et (e') peu(ven)t représenter moins de 50 % en poids de la composition globale du polymère multiphasique de l'invention, en particulier moins de 40 % en poids et plus particulièrement de 1 % à 30 % en poids.

Selon un mode de réalisation particulièrement préféré, le polymère multiphasique selon l'invention est formé :
(1) d'un polymère P1 obtenu par polymérisation à partir d'un mélange de monomères comprenant, voire étant formé :
   - de 30 % à 43 % en poids d'au moins un monomère (a) anionique présentant une fonction vinylique polymérisable,
   - de 50 % à 65 % en poids d'au moins un monomère (b) hydrophobe non ionique présentant une fonction vinylique polymérisable,
   - de 0,4 % à 5 % en poids d'au moins un monomère (c) réticulant,
   - d'au moins 0,5 % en poids d'au moins un monomère (d) associatif présentant une fonction vinylique polymérisable et une chaîne hydrocarbonée hydrophobe et
   - éventuellement d'au moins un monomère (e) non ionique additionnel, distinct du monomère (b) et
(2) d'un polymère P2 obtenu par polymérisation à partir d'un mélange de monomères comprenant, voire étant formé :
   - de 20 % à 43 % en poids d'au moins un monomère (a') anionique présentant une fonction vinylique polymérisable,
   - de 60 % à 75 % en poids d'au moins un monomère (b') hydrophobe non ionique présentant une fonction vinylique polymérisable,
   - de 0,3 % à 5 % en poids d'au moins un monomère (c') réticulant,
   - éventuellement d'au moins un monomère (d') associatif présentant une fonction vinylique polymérisable et une chaîne hydrocarbonée hydrophobe et
   - éventuellement d'au moins un monomère (e') non ionique additionnel, distinct du monomère (b').

Selon un mode de réalisation particulier, le polymère multiphasique selon l'invention est obtenu à partir d'au moins les monomères suivants :
- un ou plusieurs monomère(s) anionique(s) choisi(s) parmi l'acide acrylique et/ou l'acide méthacrylique et/ou l'un de leurs sels, en particulier l'acide méthacrylique,
- un ou plusieurs monomère(s) hydrophobe(s) non ionique(s) choisi(s) parmi l'acrylate d'éthyle, l'acrylate de butyle, le méthacrylate d'éthyle ou leurs mélanges, en particulier l'acrylate d'éthyle,
- un ou plusieurs monomère(s) réticulant(s), en particulier tel(s) que défini(s) précédemment et plus particulièrement le triacrylate de triméthylolpropane,
- un ou plusieurs monomère(s) associatif(s) de formule (II) ou (III) précitée, en particulier de formule (IV) ou (V) précitée et plus particulièrement tel(s) que décrit(s) précédemment et
- éventuellement un ou plusieurs monomère(s) additionnel(s) non ionique(s) tel(s) que défini(s) précédemment.

### Préparation du polymère multiphasique selon l'invention

Les polymères multiphasiques selon l'invention peuvent être préparés de façon séquentielle par un procédé de polymérisation radicalaire en émulsion, dispersion ou solution.

De préférence, ils sont préparés par polymérisation radicalaire en au moins deux étapes, le polymère P1 et le polymère P2 étant produits dans deux étapes de polymérisation en émulsion séquentielle, en particulier dans cet ordre P1 puis P2.

La polymérisation est conduite dans des solvants appropriés, en présence d'initiateurs connus.

A titre d'exemple, l'initiateur de polymérisation peut être un sel persulfate tel que le persulfate d'ammonium.

La polymérisation radicalaire en émulsion peut avantageusement être réalisée en présence d'au moins un tensioactif et éventuellement d'au moins un agent de transfert de chaînes, permettant de réguler la masse moléculaire des chaînes produites lors de la polymérisation.

Comme tensioactifs susceptibles d'être utilisés, on peut citer les tensioactifs anioniques tels qu'un sel d'acide gras, un sel alkylsulfate (comme le laurylsulfate de sodium), un sel d'alkyléther sulfate (comme le lauryl éther sulfate de sodium), un sel alkylbenzènesulfate (comme le dodécylbenzènesulfonate de sodium), un sel alkylphosphate ou un sel sulfosuccinate diester ou des tensioactifs non ioniques tels qu'un polyoxyéthylène alkyléther ou un ester d'acide gras polyoxyéthylène, les tensioactifs cationiques tels que les halogénures d'alkyl- et/ou aryl-ammoniums quaternaires, les tensioactifs zwitterioniques ou amphotères tels que les tensioactifs comprenant un groupe bétaïne.

Comme agents de transfert de chaînes, on peut citer, avantageusement, les composés mercaptans comprenant au moins quatre atomes de carbone tels que le butylmercaptan, le n-octylmercaptan, le n-dodécylmercaptan, le *tert*-dodécylmercaptan.

La polymérisation en émulsion est réalisée, classiquement, dans un milieu de dispersion aqueux.

Ainsi, l'invention vise plus particulièrement un procédé de préparation d'un polymère multiphasique selon l'invention, comprenant au moins les étapes consécutives suivantes :
(i) polymérisation du polymère P1 à partir d'un premier mélange de monomères comprenant :
   - au moins un monomère (a) anionique présentant une fonction vinylique polymérisable,
   - au moins un monomère (b) hydrophobe non ionique présentant une fonction vinylique polymérisable,
   - au moins un monomère (c) réticulant,
   - au moins un monomère (d) associatif présentant une fonction vinylique polymérisable et une chaîne hydrocarbonée hydrophobe et
   - éventuellement au moins un monomère (e) non ionique additionnel, distinct du monomère (b) et
(ii) polymérisation en présence du polymère P1 précédemment obtenu d'un second mélange de monomères comprenant :
   - au moins un monomère (a') anionique présentant une fonction vinylique polymérisable,
   - au moins un monomère (b') hydrophobe non ionique présentant une fonction vinylique polymérisable,
   - au moins un monomère (c') réticulant,
   - éventuellement un monomère (d') associatif présentant une fonction vinylique polymérisable et une chaîne hydrocarbonée hydrophobe et
   - éventuellement un monomère (e') non ionique additionnel, distinct du monomère (b').

D'un point de vue pratique, la première étape consiste à mettre en contact les monomères destinés à entrer dans la composition du polymère P1 avec un initiateur de polymérisation, cette mise en contact pouvant être réalisée en mode discontinu (ou mode batch) ou en mode semi-continu (la mise en contact étant réalisée sur une durée pouvant aller de plusieurs minutes à plusieurs heures).

D'un point de vue pratique, la deuxième étape (étape de réalisation du polymère P2) peut se dérouler de la façon suivante :
- une étape d'ajout des monomères destinés à entrer dans la composition du polymère P2 à un milieu de dispersion comprenant le polymère P1 déjà formé, cet ajout pouvant se faire selon un mode discontinu ou semi-continu (la mise en contact étant réalisée sur une durée pouvant aller de plusieurs minutes à plusieurs heures) et
- simultanément pour le mode semi-continu ou postérieurement à cette étape d'ajout pour le mode discontinu, une étape d'introduction d'un initiateur de polymérisation.

### Applications

Les polymères multiphasiques selon l'invention se révèlent particulièrement efficaces à titre d'agents modificateurs de rhéologie dans une large gamme de compositions aqueuses, en particulier des compositions lavantes renfermant des tensioactifs telles que des compositions de soin ou des compositions d'entretien. L'expression « compositions de soin » comprend, par exemple, des compositions cosmétiques, d'hygiène de la personne, des produits de toilette et compositions nettoyantes pour une application sur le corps (incluant la peau, les cheveux, les ongles) des humains ou animaux, par exemple des compositions de shampoing. L'expression « compositions d'entretien » inclut les compositions utilisées pour le nettoyage ou maintien des conditions sanitaires, par exemple dans la cuisine, la salle de bain, les produits détergents, les produits de lessive, etc.

L'invention concerne ainsi, selon encore un autre de ses aspects, une composition aqueuse comprenant au moins un polymère multiphasique selon l'invention ou tel qu'obtenu selon le procédé décrit ci-avant.

Le polymère multiphasique selon l'invention peut être mis en œuvre dans la composition aqueuse à raison de 0,1 % à 20 % en poids, en particulier de 0,5 % à 12 % en poids, par rapport au poids total de la composition.

Comme illustré dans les exemples qui suivent, le polymère selon l'invention permet de combiner avantageusement des performances en termes d'effet épaississant, de clarté et de propriétés suspensivantes. Autrement dit, il permet l'obtention d'une composition aqueuse présentant la viscosité souhaitée et comprenant une phase continue limpide et des particules en suspension réparties de manière homogène dans la phase continue.

L'invention concerne, ainsi, plus particulièrement l'utilisation d'un polymère multiphasique selon l'invention dans une composition aqueuse à titre d'agent épaississant et suspensif.

Elle concerne encore l'utilisation pour la préparation d'une composition aqueuse stable, comprenant une phase continue limpide et des particules en suspension réparties dans la phase continue, ayant de préférence un pH inférieur ou égal à 6, d'un polymère multiphasique tel que défini précédemment.

L'invention concerne encore un agent pour l'obtention d'une composition aqueuse stable, comprenant une phase continue limpide et des particules en suspension réparties dans la phase continue, en particulier de pH inférieur ou égal à 6, comprenant un polymère multiphasique selon l'invention.

En plus de la clarté qu'il apporte, l'agent de l'invention permet, ainsi, de maintenir en suspension les particules présentes dans la composition. L'utilisation d'une composition ainsi formulée ne nécessite donc aucune étape de mélange, même si la composition a été stockée plusieurs semaines, voire plusieurs mois.

Une composition selon l'invention peut comprendre des ingrédients classiquement mis en œuvre dans les formulations évoquées précédemment. Elle peut comprendre un ou plusieurs ingrédient(s) actif(s) (ou agent(s) actif(s)), sous toute forme que ce soit et quel que soit le domaine d'application de la composition, comme indiqué précédemment. Le ou les principe(s) actif(s) peu(ven)t être dissou(s) dans la phase continue de la composition et/ou ils peuvent être sous forme particulaire, non soluble dans la phase continue et constituer tout ou partie des particules en suspension.

Elle peut comprendre un ou plusieurs tensioactif(s), en particulier choisi(s) parmi les tensioactifs anioniques, zwitterioniques ou amphotères, cationiques ou non-ioniques et leurs mélanges.

L'invention concerne plus particulièrement une composition cosmétique aqueuse, comprenant une phase continue et des particules en suspension dans la phase continue, ladite phase continue et/ou lesdites particules comprenant et/ou consistant en un principe actif cosmétique, ladite composition comprenant un polymère multiphasique tel que défini précédemment.

A titre de principe(s) actif(s), elle peut comprendre une base lavante pour le corps et/ou les cheveux.

De manière avantageuse, l'agent modificateur de rhéologie selon l'invention permet d'accéder aux propriétés de viscosité, de clarté et d'effet suspensivant souhaitées pour une large gamme de pH, notamment lorsqu'il est formulé dans des compositions ayant un pH inférieur ou égal à 6, en particulier inférieur ou égal à 5,5, en particulier inférieur ou égal à 5, notamment compris entre 4 et 5.

De tels pH sont proches de la valeur de pH moyenne de la peau humaine. L'agent modificateur de rhéologie selon l'invention présente ainsi un intérêt majeur en cosmétique.

L'invention va maintenant être décrite au moyen des exemples suivants, donnés bien entendu à titre illustratif et non limitatif de l'invention.

### EXEMPLES

Les abréviations suivantes sont utilisées :
**AMA** : acide (méth)acrylique.
**AE** : acrylate d'éthyle.
**MA** : monomère associatif de formule (IV) dans laquelle n' et n" sont nuls, n vaut 25, R₁ représente CH₃, Z est une chaîne ramifiée comportant 16 atomes de carbone.
**TMPTA** : triacrylate de triméthylolpropane.

### Synthèse des polymères multiphasiques

Le protocole de synthèse du polymère multiphasique noté « Pol.1 » de l'exemple 1 est le suivant :
On introduit dans un réacteur de 1 L, agité et chauffé à l'aide d'un bain d'huile, 430 g d'eau et 4,65 g de lauryl éther sulfate de sodium (Texapon NSO à 28 %).
On prépare dans un bécher le pré-mélange comprenant les monomères correspondant au polymère P1. Il contient 141,0 g d'eau, 4,9 g de Texapon NSO à 28 %, 82,5 g d'AMA, 131,7 g d'AE, 19,3 g de MA et 2,1 g de TMPTA.
On prépare dans un second bécher le pré-mélange comprenant les monomères correspondant au polymère P2. Il contient 43,4 g d'eau, 1,5 g de Texapon NSO à 28 %, 26,9 g d'AMA, 54,8 g d'AE, 6,0 g de MA et 0,7 g de TMPTA.
On prépare dans un troisième bécher la solution d'initiateur de polymérisation. Elle contient 0,587 g de persulfate d'ammonium et 55 g d'eau.

On injecte dans le réacteur maintenu à 86 ± 2 °C en parallèle la solution d'initiateur de polymérisation durant 2 heures et le pré-mélange de monomères correspondant au polymère P1 durant 1 heure et 30 minutes suivi du pré-mélange de monomères correspondant au polymère P2 durant 30 minutes.

Cette étape de polymérisation est suivie de l'injection en 1 heure d'un mélange contenant 0,1 g de persulfate d'ammonium et 35 g d'eau.
L'ensemble est ensuite refroidi à température ambiante.

L'ensemble des polymères multiphasiques présentés dans les exemples qui suivent ont été synthétisés dans les conditions décrites ci-dessus, en faisant varier les compositions de monomères et les temps d'injection des pré-mélanges de monomères.

La composition du polymère P1 (respectivement du polymère P2) y est indiquée en pourcentage en poids de chacun des monomères par rapport au poids total des monomères de P1 (respectivement de P2).

### Evaluation dans une formulation aqueuse

Les polymères sont testés dans une formulation aqueuse, de composition indiquée dans le tableau 1 suivant (2 % ou 2,4 % en poids de polymère multiphasique par rapport au poids total de la composition).

**Tableau 1**

| **Composés** | **Quantité (% en poids)** |
|---|---|
| Lauryl éther sulfate de sodium | 9 |
| Cocamidopropylbétaïne | 3 |
| Polymère testé | 2 ou 2,4 |
| eau | Qsp 100 |

Le pH de la formulation est ajusté à une valeur de 4, 5 ou 6 par ajout d'acide lactique ou d'hydroxyde de sodium.

### Propriétés évaluées

Les compositions sont évaluées pour leurs propriétés de clarté, de viscosité et de performances suspensivantes.

### Clarté

La clarté de la composition est évaluée par mesure de la transmittance selon le protocole suivant :
Les mesures sont réalisées sur un Spectromètre UV Genesys 10 UV ™ (Cole Parmer), équipé de cuves Rotilabo-Einmal Kuvetten PS, 4,5 mL. De manière pratique, on préchauffe l'appareil 10 minutes avant utilisation. On réalise d'abord une première mesure au moyen d'une cuve remplie de 3,8 mL d'eau bipermutée (le « blanc »). On réalise ensuite la mesure avec une cuve remplie de 3,8 mL de la solution de composition cosmétique à tester. La transmittance est alors mesurée à la longueur d'onde de 500 nm. Plus la valeur de transmittance, exprimée en pourcentage, est élevée plus la composition cosmétique est limpide.

Comme indiqué précédemment, on estime qu'à une valeur de transmittance à 500 nm d'au moins 60 %, la composition est limpide.

### Viscosité

On mesure la viscosité desdites formulations, à l'aide d'un viscosimètre Brookfield, modèle LVT. Avant la mesure de la viscosité, on laisse chacune des formulations au repos 24 heures à 25 °C. Le mobile doit être centré par rapport à l'ouverture du flacon.

On mesure ensuite la viscosité à 6 rpm à l'aide du module approprié. On laisse tourner le viscosimètre jusqu'à ce que la viscosité soit stable.

L'agent modificateur de rhéologie doit apporter une viscosité suffisante à la formulation dans laquelle il est mis en œuvre. D'une manière générale, la viscosité souhaitée pour les formulations épaissies est supérieure à 2000 mPa.s, en particulier supérieure à 3000 mPa.s et plus particulièrement supérieure à 4000 mPa.s.

### Performances suspensivantes

Les performances suspensivantes sont évaluées par observation de la stabilité de billes de polyéthylène de 800 µm de diamètre, dans la formulation cosmétique après stockage à l'étuve à 45°C.

Dès qu'un déplacement de particules est relevé, le test est arrêté. Les résultats sont notés comme suit :

| **Temps de stabilité** | **Note de suspension** |
|---|---|
| Jusqu'à 7 jours | 1 |
| Jusqu'à 30 jours | 2 |
| Jusqu'à 60 jours | 3 |
| Jusqu'à 75 jours | 4 |
| Plus de 90 jours | 5 |

On considère que les performances suspensivantes de la formulation sont satisfaisantes pour une durée de stabilité d'au moins 75 jours, soit une note de suspension ≥ 4.

### EXEMPLE 1

### Influence de la présence du monomère réticulant dans le polymère P1 et dans le polymère P2

**Tableau 2**

| **Polymère testé** | | **Pol. 1** | **C1 (hors invention)** |
|---|---|---|---|
| **Composition P1** | **AE** | 55,92 | 56,42 |
| | **AMA** | 35,00 | 35,31 |
| | **MA** | 8,19 | 8,26 |
| | **TMPTA** | **0,89** | **0,00** |
| **Composition P2** | **AE** | 62,01 | 62,01 |
| | **AMA** | 30,50 | 30,50 |
| | **MA** | 6,76 | 6,76 |
| | **TMPTA** | 0,74 | 0,74 |
| **Composition globale** | **AE** | 57,58 | 57,95 |
| | **AMA** | 33,77 | 33,99 |
| | **MA** | 7,80 | 7,85 |
| | **TMPTA** | 0,85 | 0,20 |
| **Proportion P1** | | 72,74 | 72,56 |
| **Proportion P2** | | 27,26 | 27,44 |
| **2,4% actif, pH=5** | **Visco Brook (mPa.s)** | 17400 | 12600 |
| | **Note de suspension** | 5 | 3 |
| | **T(500nm) (%)** | 87 | 93 |

**Tableau 3**

| **Polymère testé** | | **Pol. 2** | **C2 (hors invention)** |
|---|---|---|---|
| **Composition P1** | **AE** | 55,92 | 55,92 |
| | **AMA** | 35,00 | 35,00 |
| | **MA** | 8,19 | 8,19 |
| | **TMPTA** | 0,89 | 0,89 |
| **Composition P2** | **AE** | 62,01 | 62,47 |
| | **AMA** | 30,50 | 30,72 |
| | **MA** | 6,76 | 6,81 |
| | **TMPTA** | **0,74** | **0,00** |
| **Composition globale** | **AE** | 57,58 | 57,69 |
| | **AMA** | 33,77 | 33,84 |
| | **MA** | 7,80 | 7,82 |
| | **TMPTA** | 0,85 | 0,65 |
| **Proportion P1** | | 72,74 | 72,88 |
| **Proportion P2** | | 27,26 | 27,12 |
| **Visco Brook à 2% actif (mPa.s)** | **pH=4** | 8400 | 7100 |
| | **pH=5** | 7400 | 6500 |
| | **pH=6** | 5800 | 4900 |
| **Note de suspension à 2 % actif** | **pH=4** | 5 | 3 |
| | **pH=5** | 5 | 2 |
| | **pH=6** | 4 | 2 |
| **T(500nm) à 2% actif (%)** | **pH=4** | 82 | 81 |
| | **pH=5** | 84 | 85 |
| | **pH=6** | 91 | 90 |

Les résultats présentés dans les tableaux 2 et 3 montrent que la présence du monomère réticulant à la fois dans le polymère P1 et dans le polymère P2 permet d'accéder à une viscosité et des propriétés suspensivantes améliorées, ceci en conservant les propriétés de clarté souhaitées.

### EXEMPLE 2

### Influence de la présence du monomère associatif dans le polymère multiphasique

**Tableau 4**

| **Polymère testé** | | **Pol. 2** | **C3 (hors invention)** | **Pol. 3** | **Pol. 4** | **C4 (hors invention)** |
|---|---|---|---|---|---|---|
| **Composition P1** | **AE** | 55,92 | 60,91 | 55,92 | 54,53 | 66,58 |
| | **AMA** | 35,00 | 38,12 | 35,00 | 34,14 | 32,36 |
| | **MA** | **8,19** | **0,00** | **8,19** | **10,46** | **0,00** |
| | **TMPTA** | 0,89 | 0,97 | 0,89 | 0,87 | 1,06 |
| **Composition P2** | **AE** | 62,01 | 63,30 | 66,50 | 66,50 | 43,44 |
| | **AMA** | 30,50 | 35,95 | 32,71 | 32,71 | 35,99 |
| | **MA** | **6,76** | **0,00** | **0,00** | **0,00** | **20,05** |
| | **TMPTA** | 0,74 | 0,75 | 0,79 | 0,79 | 0,52 |
| **Composition globale** | **AE** | 57,58 | 61,59 | 58,66 | 57,57 | 57,58 |
| | **AMA** | 33,77 | 37,50 | 34,41 | 33,77 | 33,77 |
| | **MA** | 7,80 | 0,00 | 6,07 | 7,80 | 7,80 |
| | **TMPTA** | 0,85 | 0,91 | 0,86 | 0,85 | 0,85 |
| **Proportion P1** | | 72,74 | 71,44 | 74,11 | 74,58 | 61,09 |
| **Proportion P2** | | 27,26 | 28,56 | 25,90 | 25,42 | 38,91 |
| **Visco Brook à 2% actif (mPa.s)** | **pH=4** | 8400 | 2800 | 7300 | 7200 | 12200 |
| | **pH=5** | 7400 | 2400 | 6100 | 6200 | 10800 |
| **Note de suspension à 2% actif** | **pH=4** | 5 | 1 | 4 | 4 | 1 |
| | **pH=5** | 4 | 1 | 4 | 4 | 1 |
| **T(500nm) à 2% actif (%)** | **pH=4** | 82 | 85 | 82 | 81 | 93 |
| | **pH=5** | 84 | 85 | 83 | 81 | 94 |

Il ressort des résultats présentés dans le tableau 4 que l'absence de monomère associatif dans le polymère P1 ne permet pas d'accéder à de bonnes performances suspensivantes. En revanche, la présence de monomère associatif selon l'invention dans le polymère P1, et éventuellement, en outre, dans le polymère P2, du polymère multiphasique, conduit à de bonnes propriétés à la fois en termes de viscosité, de transmittance et de performances suspensivantes.

### EXEMPLE 3

### Influence de la structure multiphasique selon l'invention

Le polymère C5 a été synthétisé par polymérisation radicalaire simple en émulsion (procédé non séquentiel).

**Tableau 5**

| **Polymère testé** | | **Pol. 5** | **C5 (hors invention)** |
|---|---|---|---|
| **Composition P1** | **AE** | 55,92 | 60,44 |
| | **AMA** | 35,00 | 30,91 |
| | **MA** | 8,19 | 7,80 |
| | **TMPTA** | 0,89 | 0,85 |
| **Composition P2** | **AE** | 72,50 | |
| | **AMA** | 20,00 | |
| | **MA** | 6,76 | |
| | **TMPTA** | 0,74 | |
| **Composition globale** | **AE** | 60,44 | 60,44 |
| | **AMA** | 30,91 | 30,91 |
| | **MA** | 7,80 | 7,80 |
| | **TMPTA** | 0,85 | 0,85 |
| **Proportion P1** | | 72,74 | 100,00 |
| **Proportion P2** | | 27,26 | 0,00 |
| **2,4% actif, pH=5** | **Visco Brook (mPa.s)** | 9400 | 11100 |
| | **Note de suspension** | 5 | 3 |
| | **T(500nm) (%)** | 84 | 92 |

Ces résultats montrent que la mise en œuvre d'un polymère à structure multiphasique selon l'invention, comparativement à un polymère simple, permet d'accéder à des performances suspensivantes améliorées de la formulation, ceci en conservant des propriétés de viscosité et de transmittance satisfaisantes.

### EXEMPLE 4

### Influence du ratio polymère Pl/polymère P2

**Tableau 6**

| **Polymère testé** | | **Pol. 6** | **Pol 7** | **C6 (hors invention)** |
|---|---|---|---|---|
| **Composition P1** | **AE** | 55,92 | 55,92 | 55,92 |
| | **AMA** | 35,00 | 35,00 | 35,00 |
| | **MA** | 8,19 | 8,19 | 8,19 |
| | **TMPTA** | 0,89 | 0,89 | 0,89 |
| **Composition P2** | **AE** | 62,01 | 62,01 | 62,01 |
| | **AMA** | 30,50 | 30,49 | 30,50 |
| | **MA** | 6,76 | 6,76 | 6,76 |
| | **TMPTA** | 0,74 | 0,74 | 0,74 |
| **Composition globale** | **AE** | 57,58 | 58,17 | 60,46 |
| | **AMA** | 33,77 | 33,33 | 31,64 |
| | **MA** | 7,80 | 7,66 | 7,12 |
| | **TMPTA** | 0,85 | 0,83 | 0,78 |
| **Proportion P1** | | 72,74 | 62,98 | 25,32 |
| **Proportion P2** | | 27,26 | 37,02 | 74,68 |
| **Visco Brook à 2% actif (mPa.s)** | **pH=4** | 9300 | 8500 | 7600 |
| | **pH=5** | 8000 | 7200 | 6300 |
| **Note de suspension à 2 % actif** | **pH=4** | 5 | 5 | 3 |
| | **pH=5** | 5 | 5 | 3 |
| **T(500nm) à 2% actif (%)** | **pH=4** | 84 | 83 | 87 |
| | **pH=5** | 83 | 84 | 87 |

## Revendications

1. Polymère multiphasique comprenant de 45 % à 95 % en poids d'un premier polymère P1 et de 5 % à 55 % en poids d'un second polymère P2, lesdits polymères P1 et P2 étant de compositions distinctes,
(1) ledit polymère P1 étant obtenu par polymérisation à partir d'un mélange de monomères, distincts les uns des autres, comprenant :
- au moins un monomère (a) anionique présentant une fonction vinylique polymérisable,
- au moins un monomère (b) hydrophobe non ionique présentant une fonction vinylique polymérisable,
- au moins un monomère (c) réticulant,
- au moins un monomère (d) associatif présentant une fonction vinylique polymérisable et une chaîne hydrocarbonée hydrophobe et
- éventuellement au moins un monomère (e) non ionique additionnel, distinct du monomère (b),
(2) ledit polymère P2 étant obtenu par polymérisation à partir d'un mélange de monomères, distincts les uns des autres, comprenant :
- au moins un monomère (a') anionique présentant une fonction vinylique polymérisable,
- au moins un monomère (b') hydrophobe non ionique présentant une fonction vinylique polymérisable,
- au moins un monomère (c') réticulant,
- éventuellement au moins un monomère (d') associatif présentant une fonction vinylique polymérisable et une chaîne hydrocarbonée hydrophobe et
- éventuellement au moins un monomère (e') non ionique additionnel, distinct du monomère (b').

2. Polymère selon la revendication précédente, dans lequel :
• le rapport pondéral polymère P1/polymère P2 est compris entre 45/55 et 95/5, en particulier entre 60/40 et 95/5 ou
• le ou lesdits monomère(s) anionique(s) est(sont) choisi(s) parmi les monomères d'acide acrylique et/ou d'acide méthacrylique et/ou l'un de leurs sels ou
• le ou lesdits monomère(s) anionique(s) (a) représente(nt) de 20 % à 53 % en poids, en particulier de 25 % à 48 % en poids et plus particulièrement de 30 % à 43 % en poids, par rapport au poids total de monomères formant le polymère P1 ou
• le ou lesdits monomère(s) anionique(s) (a') représente(nt) de 10 % à 53 % en poids, en particulier de 15 % à 48 % en poids et plus particulièrement de 20 % à 43 % en poids, par rapport au poids total de monomères formant le polymère P2 ou
• le ou lesdits monomère(s) hydrophobe(s) non ionique(s) est(sont) choisi(s) parmi les acrylates d'alkyle en C₁-C₈ ou les méthacrylates d'alkyle en C₁-C₈ tels que l'acrylate de méthyle, l'acrylate d'éthyle, l'acrylate de butyle, l'acrylate de 2-éthyle-hexyle, le méthacrylate de méthyle, le méthacrylate d'éthyle, le méthacrylate de butyle et leurs mélanges ou
• le ou lesdits monomère(s) hydrophobe(s) non ionique(s) (b) représente(nt) de 40 % à 75 % en poids, en particulier de 45 % à 70 % en poids et plus particulièrement de 50 % à 65 % en poids, par rapport au poids total de monomères formant le polymère P1 ou
• le ou lesdits monomère(s) hydrophobe(s) non ionique(s) (b') représente(nt) de 50 % à 85 % en poids, en particulier de 55 % à 80 % en poids et plus particulièrement de 60 % à 75 % en poids, par rapport au poids total de monomères formant le polymère P2.

3. Polymère selon l'une quelconque des revendications précédentes dans lequel le rapport pondéral monomères hydrophobes non ioniques (b')/monomères anioniques (a') de la composition du polymère P2 est compris entre 60/40 et 85/15, en particulier entre 65/35 et 80/20.

4. Polymère selon l'une quelconque des revendications précédentes, dans lequel le ou lesdits monomère(s) réticulant(s) est(sont) choisi(s) parmi les réticulants trifonctionnels, en particulier le triacrylate de triméthylolpropane (TMPTA).

5. Polymère selon l'une quelconque des revendications précédentes, dans lequel la teneur en monomère(s) réticulant(s) (c) dans la composition du polymère P1 est supérieure ou égale à 0,3 % en poids, par rapport au poids total des monomères formant le polymère P1, en particulier supérieure ou égale à 0,4 % en poids et de préférence comprise entre 0,4 % et 5 % en poids.

6. Polymère selon l'une quelconque des revendications précédentes, dans lequel la teneur en monomère(s) réticulant(s) (c') dans la composition du polymère P2 peut être plus particulièrement supérieure ou égale à 0,2 % en poids, par rapport au poids total des monomères formant le polymère P2, en particulier supérieure ou égale à 0,3 % et notamment comprise entre 0,3 % et 5 % en poids.

7. Polymère selon l'une quelconque des revendications précédentes, dans lequel le ou lesdits monomère(s) associatif(s) est(sont) choisi(s) parmi les monomères de formule (I) suivante :
T-[(EO)ₙ(PO)_{n'}(BO)_{n"}]-Z (I)
dans laquelle :
- T représente une extrémité permettant la copolymérisation du monomère associatif,
- [(EO)ₙ(PO)_{n'}(BO)_{n"}] représente une chaîne polyalkoxylée constituée d'unités alkoxylées, réparties en blocs, alternées ou statistiques, choisies parmi les unités éthoxylées EO, les unités propoxylées PO et les unités butoxylées BO,
- n, n', n" représentent, indépendamment les uns des autres, 0 ou un nombre entier variant de 1 à 150 et
- Z représente une chaîne grasse, linéaire ou ramifiée, d'au moins 12 atomes de carbone et comportant éventuellement un ou plusieurs groupe(s) cyclique(s) de 5 à 7 chaînons, saturés, partiellement insaturés ou aromatiques, lesdits groupes pouvant être éventuellement substitués.

8. Polymère selon l'une quelconque des revendications précédentes, dans lequel le ou lesdits monomère(s) associatif(s) est(sont) mis en œuvre à raison d'au moins 0,5 % en poids, en particulier de 0,5 % à 12 % en poids, par rapport au poids total de monomères formant le polymère P1.

9. Polymère selon l'une quelconque des revendications précédentes, dans lequel les monomères additionnels non ioniques (e) et (e') sont choisis parmi :
- l'acide 2-acrylamido-2-méthylpropane sulfonique (AMPS),
- les télomères insaturés de l'acide acrylique,
- les monomères de formule (el) : 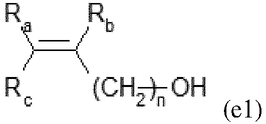 dans laquelle :
- Rₐ, R_{b} et R_{c} représentent, indépendamment les uns des autres, H ou CH3,
- n est un entier égal à 1 ou à 2 et
- les monomères de formule (e2) : 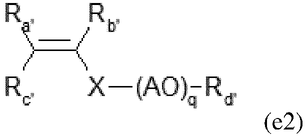 dans laquelle :
- R_{a'}, R_{b'}, R_{c'} et R_{d'} représentent, indépendamment les uns des autres, H ou CH3,
- X représente (C=O) ou (CH₂)ᵣ avec r = 0, 1 ou 2,
- (AO) représente une chaîne polyalkoxylée constituée d'unités alkoxylées, réparties en blocs, alternées ou statistiques, choisies parmi les unités éthoxylées EO, les unités propoxylées PO et les unités butoxylées BO et
- q représente 0 ou un nombre entier variant de 1 à 150.

10. Polymère selon l'une quelconque des revendications précédentes, ledit polymère étant formé :
(1) d'un polymère P1 obtenu par polymérisation à partir d'un mélange de monomères comprenant, voire étant formé :
- de 30 % à 43 % en poids d'au moins un monomère (a) anionique présentant une fonction vinylique polymérisable,
- de 50 % à 65 % en poids d'au moins un monomère (b) hydrophobe non ionique présentant une fonction vinylique polymérisable,
- de 0,4 % à 5 % en poids d'au moins un monomère (c) réticulant,
- d'au moins 0,5 % en poids d'au moins un monomère (d) associatif présentant une fonction vinylique polymérisable et une chaîne hydrocarbonée hydrophobe et
- éventuellement d'au moins un monomère (e) non ionique additionnel, distinct du monomère (b) et
(2) d'un polymère P2 obtenu par polymérisation à partir d'un mélange de monomères comprenant, voire étant formé :
- de 20 % à 43 % en poids d'au moins un monomère (a') anionique présentant une fonction vinylique polymérisable,
- de 60 % à 75 % en poids d'au moins un monomère (b') hydrophobe non ionique présentant une fonction vinylique polymérisable,
- de 0,3 % à 5 % en poids d'au moins un monomère (c') réticulant,
- éventuellement d'au moins un monomère (d') associatif présentant une fonction vinylique polymérisable et une chaîne hydrocarbonée hydrophobe et
- éventuellement au moins un monomère (e') non ionique additionnel, distinct du monomère (b').

11. Procédé de préparation d'un polymère multiphasique tel que défini selon l'une quelconque des revendications 1 à 10, comprenant au moins les étapes consécutives suivantes :
(i) polymérisation du polymère P1 à partir d'un premier mélange de monomères, distincts les uns des autres, comprenant :
- au moins un monomère (a) anionique présentant une fonction vinylique polymérisable,
- au moins un monomère (b) hydrophobe non ionique présentant une fonction vinylique polymérisable,
- au moins un monomère (c) réticulant,
- au moins un monomère (d) associatif présentant une fonction vinylique polymérisable et une chaîne hydrocarbonée hydrophobe et
- éventuellement au moins un monomère (e) non ionique additionnel, distinct du monomère (b) et
(ii) polymérisation en présence du polymère P1 précédemment obtenu d'un second mélange de monomères, distincts les uns des autres, comprenant :
- au moins un monomère (a') anionique présentant une fonction vinylique polymérisable,
- au moins un monomère (b') hydrophobe non ionique présentant une fonction vinylique polymérisable,
- au moins un monomère (c') réticulant,
- éventuellement un monomère (d') associatif présentant une fonction vinylique polymérisable et une chaîne hydrocarbonée hydrophobe et
- éventuellement au moins un monomère (e') non ionique additionnel, distinct du monomère (b').

12. Composition aqueuse comprenant au moins un polymère multiphasique tel que défini selon l'une quelconque des revendications 1 à 10 ou tel qu'obtenu selon le procédé de la revendication 11.

13. Composition selon la revendication précédente, ladite composition présentant une valeur de pH inférieure ou égale à 6, en particulier inférieure ou égale à 5,5, et plus particulièrement inférieure ou égale à 5.

14. Composition selon la revendication 12 ou 13, comprenant de 0,1 % à 20 % en poids, en particulier de 0,5 % à 12 % en poids de polymère(s) multiphasique(s) par rapport à son poids total.

15. Utilisation d'un polymère multiphasique tel que défini selon l'une quelconque des revendications 1 à 10 ou tel qu'obtenu selon le procédé de la revendication 11, à titre d'agent épaississant et suspensif.

## Patentansprüche

1. Mehrphasiges Polymer bestehend zu 45 Gew.-% bis 95 Gew.-% aus einem ersten Polymer P1 und zu 5 Gew.-% bis 55 Gew.-% aus einem zweiten Polymer P2, wobei diese Polymere P1 und P2 unterschiedliche Zusammensetzungen aufweisen,
(1) wobei das Polymer P1 durch Polymerisation aus einer Mischung von unterschiedlichen Monomeren herstellt wird, bestehend aus:
- mindestens einem anionischen Monomer (a) mit einer polymerisierbaren funktionellen Vinylgruppe,
- mindestens einem nicht-ionischen hydrophoben Monomer (b) mit einer polymerisierbaren funktionellen Vinylgruppe,
- mindestens einem vernetzten Monomer (c),
- mindestens einem assoziativen Monomer (d) mit einer polymerisierbaren funktionellen Vinylgruppe und einer hydrophoben Kohlenwasserstoffkette und
- gegebenenfalls mindestens einem zusätzlichen nicht-ionischen Monomer (e), das mit Monomer (b) nicht identisch ist,
(2) wobei das Polymer P2 durch Polymerisation aus einer Mischung von unterschiedlichen Monomeren herstellt wird, bestehend aus:
- mindestens einem anionischen Monomer (a') mit einer polymerisierbaren funktionellen Vinylgruppe,
- mindestens einem nicht-ionischen hydrophoben Monomer (b') mit einer polymerisierbaren funktionellen Vinylgruppe,
- mindestens einem vernetzten Monomer (c'),
- mindestens einem assoziativen Monomer (d') mit einer polymerisierbaren funktionellen Vinylgruppe und einer hydrophoben Kohlenwasserstoffkette und
- gegebenenfalls mindestens einem zusätzlichen nicht-ionischen Monomer (e'), das mit Monomer (b') nicht identisch ist.

2. Polymer nach dem vorstehenden Anspruch, wobei :
• das Gewichtsverhältnis von Polymer Pl/Polymer P2 zwischen 45/55 und 95/5, insbesondere zwischen 60/40 und 95/5, liegt oder
• dieses oder diese anionische(n) Monomer(e) ausgewählt ist(sind) aus den Acrylsäure- und/oder Methacrylsäuremonomeren und/oder einem ihrer Salze oder
• dieses oder diese anionische(n) Monomer(e) (a) 20 Gew.-% bis 53 Gew.-%, insbesondere 25 Gew.-% bis 48 Gew.-% und ganz besonders 30 Gew.-% bis 43 Gew.-%, bezogen auf das Gesamtgewicht an Monomeren im Polymer P1, ausmacht bzw. ausmachen oder
• dieses oder diese anionische(n) Monomer(e) (a') 10 Gew.-% bis 53 Gew.-%, insbesondere 15 Gew.-% bis 48 Gew.-% und ganz besonders 20 Gew.-% bis 43 Gew.-%, bezogen auf das Gesamtgewicht an Monomeren im Polymer P2, ausmacht bzw. ausmachen oder
• dieses oder diese nicht-ionische(n) hydrophobe(n) Monomer(e) ausgewählte ist(sind) aus den C₁-C₈ Alkylacrylaten oder den C₁-C₈ Alkylmethacrylaten, wie Methylacrylat, Ethylacrylat, Butylacrylat, 2-Ethylhexylacrylat, Methylmethacrylat, Ethylmethacrylat, Butylmethacryalt und deren Mischungen oder
• dieses oder diese nicht-ionische(n) hydrophobe(n) Monomer(e) (b) 40 Gew.-% bis 75 Gew.-%, insbesondere 45 Gew.-% bis 70 Gew.-% und ganz besonders 50 Gew.-% bis 65 Gew.-%, bezogen auf das Gesamtgewicht an Monomeren im Polymer P1, ausmacht bzw. ausmachen oder
• dieses oder diese nicht-ionische(n) hydrophobe(n) Monomer(e) (b') 50 Gew.-% bis 85 Gew.-%, insbesondere 55 Gew.-% bis 80 Gew.-% und ganz besonders 60 Gew.-% bis 75 Gew.-%, bezogen auf das Gesamtgewicht an Monomeren im Polymer P2, ausmacht bzw. ausmachen.

3. Polymer nach einem beliebigen der vorstehenden Ansprüche, wobei das Gewichtsverhältnis von nicht-ionischen hydrophoben Monomeren (b')/anionischen Monomeren (a') in der Zusammensetzung von Polymer P2 zwischen 60/40 und 85/15 und insbesondere zwischen 65/35 und 80/20 liegt.

4. Polymer nach einem beliebigen der vorstehenden Ansprüche, wobei dieses oder diese vernetzte(n) Monomer(e) ausgewählt ist(sind) aus trifunktionellen Vernetzungsmitteln und insbesondere Trimethylpropantriacrylat (TMPTA).

5. Polymer nach einem beliebigen der vorstehenden Ansprüche, wobei der Gehalt an vernetzten Monomeren (c) in der Zusammensetzung von Polymer P1 größer oder gleich 0,3 Gew.-%, bezogen auf das Gesamtgewicht an Monomeren im Polymer P1, insbesondere größer oder gleich 0,4 Gew.-%, ist und vorzugsweise zwischen 0,4 Gew.-% und 5 Gew.-% liegt.

6. Polymer nach einem beliebigen der vorstehenden Ansprüche, wobei der Gehalt an vernetzten Monomeren (c') in der Zusammensetzung von Polymer P2 größer oder gleich 0,2 Gew.-%, bezogen auf das Gesamtgewicht an Monomeren im Polymer P2, insbesondere größer oder gleich 0,3 Gew.-%, sein kann und vorzugsweise zwischen 0,3 Gew.-% und 5 Gew.-% liegt.

7. Polymer nach einem beliebigen der vorstehenden Ansprüche, wobei dieses oder diese assoziative(n) Monomer(e) ausgewählt ist(sind) aus den Monomeren mit folgender Formel (I):
T-[(EO)ₙ(PO)_{n'}(BO)_{n"}]-Z (I)
wobei:
- T einen Endabschnitt darstellt, der die Copolymerisation des assoziativen Monomers ermöglicht,
- [(EO)ₙ(PO)_{n'}(BO)_{n"}] eine polyalkoxylierte Kette aus alkoxylierten Gruppen darstellt, die in alternierende oder statistische Blöcke aufgeteilt sind, die ausgewählt sind aus ethoxylierten EO-Gruppen, propoxylierten PO-Gruppen und butoxylierten BO-Gruppen,
- n, n', n" unabhängig voneinander 0 oder eine Ganzzahl zwischen 1 und 150 darstellen und
- Z eine lineare oder verzweigte Fettkette darstellt, die mindestens 12 Kohlenstoffatome aufweist undgegebenenfalls eine oder mehrere 5- bis 7-gliedrige zyklische Gruppe(n) umfasst, die gesättigt, teilweise ungesättigt oder aromatisch sind, wobei diese Gruppen gegebenenfalls substituiert werden können.

8. Polymer nach einem beliebigen der vorstehenden Ansprüche, wobei dieses oder diese assoziative(n) Monomer(e) zu mindestens 0,5 Gew.-%, insbesondere 0,5 Gew.-% bis 12 Gew.-%, bezogen auf das Gesamtgewicht an Monomeren im Polymer P1, eingesetzt wird(werden).

9. Polymer nach einem beliebigen der vorstehenden Ansprüche, wobei die zusätzlichen nicht-ionischen Monomere (e) und (e') ausgewählt sind aus:
- 2-Acrylamido-2-methylpropansulfonsäure (AMPS),
- den ungesättigten Telomeren von Acrylsäure,
- den Monomeren mit der Formel (e1): 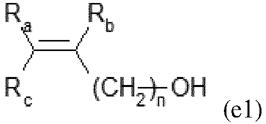 wobei:
- Rₐ, R_{b} und R_{c} unabhängig voneinander H oder CH3 darstellen,
- n einer Ganzzahl 1 oder 2 entspricht, und
- den Monomeren mit der Formel (e2): 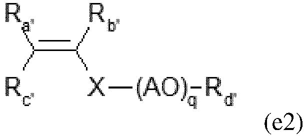 wobei:
- R_{a'}, R_{b'}, R_{c'} und R_{d'} unabhängig voneinander H oder CH3 darstellen,
- X (C=O) oder (CH₂)ᵣ mit r = 0, 1 oder 2 entspricht,
- (AO) eine polyalkoxylierte Kette aus alkoxylierten Gruppen darstellt, die in alternierende oder statistische Blöcke aufgeteilt sind, die ausgewählt sind aus ethoxylierten EO-Gruppen, propoxylierten PO-Gruppen und butoxylierten BO-Gruppen, und
- q für 0 oder eine Ganzzahl zwischen 1 und 150 steht.

10. Polymer nach einem beliebigen der vorstehenden Ansprüche, wobei dieses Polymer zusammengesetzt ist aus:
(1) einem Polymer P1, das durch Polymerisation aus einer Mischung von Monomeren hergestellt ist, zusammengesetzt aus:
- 30 Gew.-% bis 43 Gew.-% mindestens eines anionischen Monomers (a) mit einer polymerisierbaren funktionellen Vinylgruppe,
- 50 Gew.-% bis 65 Gew.-% mindestens eines nicht-ionischen hydrophoben Monomers (b) mit einer polymerisierbaren funktionellen Vinylgruppe,
- 0,4 Gew.-% bis 5 Gew.-% mindestens eines vernetzten Monomers (c),
- mindestens 0,5 Gew.-% mindestens eines assoziativen Monomers (d) mit einer polymerisierbaren funktionellen Vinylgruppe und einer hydrophoben Kohlenwasserstoffkette und
- gegebenenfalls mindestens einem zusätzlichen nicht-ionischen Monomer (e), das mit Monomer (b) nicht identisch ist, und
(2) einem Polymer P2, das durch Polymerisation aus einer Mischung von Monomeren hergestellt ist, zusammengesetzt aus:
- 20 Gew.-% bis 43 Gew.-% mindestens eines anionischen Monomers (a') mit einer polymerisierbaren funktionellen Vinylgruppe,
- 60 Gew.-% bis 75 Gew.-% mindestens eines nicht-ionischen hydrophoben Monomers (b') mit einer polymerisierbaren funktionellen Vinylgruppe,
- 0,3 Gew.-% bis 5 Gew.-% mindestens eines vernetzten Monomers (c'),
- gegebenenfalls mindestens einem assoziativen Monomer (d') mit einer polymerisierbaren funktionellen Vinylgruppe und einer hydrophoben Kohlenwasserstoffkette und
- gegebenenfalls mindestens einem zusätzlichen nicht-ionischen Monomer (e'), das mit Monomer (b') nicht identisch ist.

11. Verfahren zur Herstellung eines mehrphasigen Polymers gemäß einem beliebigen der vorstehenden Ansprüche 1 bis 10, das mindestens folgende aufeinanderfolgende Schritte umfasst:
(i) Polymerisation des Polymers P1 aus einer ersten Mischung von unterschiedlichen Monomeren bestehend aus:
- mindestens einem anionischen Monomer (a) mit einer polymerisierbaren funktionellen Vinylgruppe,
- mindestens einem nicht-ionischen hydrophoben Monomer (b) mit einer polymerisierbaren funktionellen Vinylgruppe,
- mindestens einem vernetzten Monomer (c),
- mindestens einem assoziativen Monomer (d) mit einer polymerisierbaren funktionellen Vinylgruppe und einer hydrophoben Kohlenwasserstoffkette und
- gegebenenfalls mindestens einem zusätzlichen nicht-ionischen Monomer (e), das mit Monomer (b) nicht identisch ist, und
(ii) Polymerisation in Anwesenheit von Polymer P1, das zuvor aus einer zweiten Mischung von unterschiedlichen Monomeren hergestellt wurde, bestehend aus:
- mindestens einem anionischen Monomer (a') mit einer polymerisierbaren funktionellen Vinylgruppe,
- mindestens einem nicht-ionischen hydrophoben Monomer (b') mit einer polymerisierbaren funktionellen Vinylgruppe,
- mindestens einem vernetzten Monomer (c'),
- gegebenenfalls einem assoziativen Monomer (d') mit einer polymerisierbaren funktionellen Vinylgruppe und einer hydrophoben Kohlenwasserstoffkette und
- gegebenenfalls mindestens einem zusätzlichen nicht-ionischen Monomer (e'), das mit Monomer (b') nicht identisch ist.

12. Wässrige Zusammensetzung aus mindestens einem mehrphasigen Polymer gemäß einem beliebigen der vorstehenden Ansprüche 1 bis 10 oder gemäß dem Verfahren aus Anspruch 11.

13. Zusammensetzung gemäß vorstehendem Anspruch, wobei diese Zusammensetzung einen pH-Wert von kleiner oder gleich 6, insbesondere kleiner oder gleich 5,5 und ganz besonders kleiner oder gleich 5 aufweist.

14. Zusammensetzung nach Anspruch 12 oder 13 aus 0,1 Gew.-% bis 20 Gew.-%, insbesondere 0,5 Gew.-% bis 12 Gew.-%, von mehrphasigen Polymeren bezogen auf deren Gesamtgewicht.

15. Verwendung eines mehrphasigen Polymers gemäß einem beliebigen der vorstehenden Ansprüche 1 bis 10 oder gemäß dem Verfahren aus Anspruch 11 als Verdickungs- und Supensionsmittel.

## Claims

1. A multiphasic polymer comprising from 45% to 95% by weight of a first polymer P1 and from 5% to 55% by weight of a second polymer P2, said polymers P1 and P2 being of distinct compositions,
(1) said polymer P1 being obtained by polymerization from a mixture of monomers, distinct from each other, comprising:
- at least one anionic monomer (a) having a polymerizable vinyl group,
- at least one nonionic hydrophobic monomer (b) having a polymerizable vinyl group,
- at least one cross-linking monomer (c),
- at least one associative monomer (d) having a polymerizable vinyl group and a hydrophobic hydrocarbon chain and
- optionally at least one additional nonionic monomer (e), distinct from the monomer (b),
(2) said polymer P2 being obtained by polymerization from a mixture of monomers, distinct from each other, comprising:
- at least one anionic monomer (a') having a polymerizable vinyl group,
- at least one nonionic hydrophobic monomer (b') having a polymerizable vinyl group,
- at least one cross-linking monomer (c'),
- optionally at least one associative monomer (d') having a polymerizable vinyl group and a hydrophobic hydrocarbon chain and
- optionally at least one additional nonionic monomer (e'), distinct from the monomer (b').

2. The polymer according to the preceding claim, in which
• the polymer P1/polymer P2 weight ratio is between 45/55 and 95/5, in particular between 60/40 and 95/5 or
• said anionic monomer(s) is(are) chosen from the monomers of acrylic acid and/or of methacrylic acid and/or one of their salts or
• said anionic monomer(s) (a) represent(s) from 20% to 53% by weight, in particular from 25% to 48% by weight and more particularly from 30% to 43% by weight, based on the total weight of monomers constituting the polymer P1 or
• said anionic monomer(s) (a') represent(s) from 10% to 53% by weight, in particular from 15% to 48% by weight and more particularly from 20% to 43% by weight, based on the total weight of monomers constituting the polymer P2 or
• said nonionic hydrophobic monomer(s) is(are) chosen from C₁-C₈ alkyl acrylates or C₁-C₈ alkyl methacrylates, such as methyl acrylate, ethyl acrylate, butyl acrylate, 2-ethylhexyl acrylate, methyl methacrylate, ethyl methacrylate, butyl methacrylate and their mixtures or
• said nonionic hydrophobic monomer(s) (b) represent(s) from 40% to 75% by weight, in particular from 45% to 70% by weight and more particularly from 50% to 65% by weight, based on the total weight of monomers constituting the polymer P1 or
• said nonionic hydrophobic monomer(s) (b') represent(s) from 50% to 85% by weight, in particular from 55% to 80% by weight and more particularly from 60% to 75% by weight, based on the total weight of monomers constituting the polymer P2.

3. The polymer according to any one of the preceding claims, in which the nonionic hydrophobic monomers (b')/anionic monomers (a') weight ratio of the composition of the polymer P2 is between 60/40 and 85/15, in particular between 65/35 and 80/20.

4. The polymer according to any one of the preceding claims, in which said cross-linking monomer(s) is(are) chosen from trifunctional cross-linking monomers, in particular trimethylolpropane triacrylate (TMPTA).

5. The polymer according to any one of the preceding claims, in which the content of cross-linking monomer(s) (c) in the composition of the polymer P1 is greater than or equal to 0.3% by weight, based on the total weight of the monomers constituting the polymer P1, in particular greater than or equal to 0.4% by weight and preferably between 0.4% and 5% by weight.

6. The polymer according to any one of the preceding claims, in which the content of cross-linking monomer(s) (c') in the composition of the polymer P2 may be more particularly greater than or equal to 0.2% by weight, based on the total weight of the monomers constituting the polymer P2, in particular greater than or equal to 0.3% and in particular between 0.3% and 5% by weight.

7. The polymer according to any one of the preceding claims, in which said associative monomer(s) is(are) chosen from the monomers of following formula (I):
T-[(EO)ₙ(PO)_{n'}(BO)_{n"}]-Z (I)
in which:
- T represents an end which makes possible the copolymerization of the associative monomer,
- [(EO)ₙ(PO)_{n'}(BO)_{n"}] represents a polyalkoxylated chain consisting of alkoxylated units, distributed in blocks, alternating or random, chosen from ethoxylated units EO, propoxylated units PO and butoxylated units BO,
- n, n' and n" represent, independently of one another, 0 or an integer varying from 1 to 150 and
- Z represents a linear or branched fatty chain of at least 12 carbon atoms and optionally comprising one or more 5- to 7-membered cyclic group(s) which are saturated, partially unsaturated or aromatic; said groups may be optionally substituted.

8. The polymer according to any one of the preceding claims, in which said associative monomer(s) is(are) used in a proportion of at least 0.5% by weight, in particular from 0.5% to 12% by weight, based on the total weight of monomers constituting the polymer P1.

9. The polymer according to any one of the preceding claims, in which the additional nonionic monomers (e) and (e') are chosen from:
- 2-acrylamido-2-methylpropanesulfonic acid (AMPS),
- unsaturated telomers of acrylic acid,
- the monomers of formula (e1): 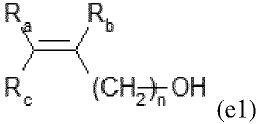 in which:
- Rₐ, R_{b} and R_{c} represent, independently of one another, H or CH3,
- n is an integer equal to 1 or 2 and
- the monomers of formula (e2): 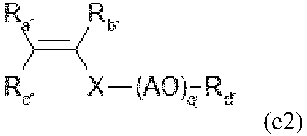 in which:
- R_{a'}, R_{b'}, R_{c'} and R_{d'} represent, independently of one another, H or CH3,
- X represents (C=O) or (CH₂)ᵣ with r = 0, 1 or 2,
- (AO) represents a polyalkoxylated chain consisting of alkoxylated units, distributed in blocks, alternating or random, chosen from ethoxylated units EO, propoxylated units PO and butoxylated units BO and
- q represents 0 or an integer varying from 1 to 150.

10. The polymer according to any one of the preceding claims, said polymer being constituted:
(1) of a polymer P1 obtained by polymerization from a mixture of monomers comprising, indeed even being constituted of:
- from 30% to 43% by weight of at least one anionic monomer (a) having a polymerizable vinyl group,
- from 50% to 65% by weight of at least one nonionic hydrophobic monomer (b) having a polymerizable vinyl group,
- from 0.4% to 5% by weight of at least one cross-linking monomer (c),
- at least 0.5% by weight of at least one associative monomer (d) having a polymerizable vinyl group and a hydrophobic hydrocarbon chain and
- optionally at least one additional nonionic monomer (e), distinct from the monomer (b) and
(2) of a polymer P2 obtained by polymerization from a mixture of monomers comprising, indeed even being constituted of:
- from 20% to 43% by weight of at least one anionic monomer (a') having a polymerizable vinyl group,
- from 60% to 75% by weight of at least one nonionic hydrophobic monomer (b') having a polymerizable vinyl group,
- from 0.3% to 5% by weight of at least one cross-linking monomer (c'),
- optionally at least one associative monomer (d') having a polymerizable vinyl group and a hydrophobic hydrocarbon chain and
- optionally at least one additional nonionic monomer (e'), distinct from the monomer (b').

11. A method for the preparation of a multiphasic polymer as defined according to any one of claims 1 to 10, comprising at least the following consecutive steps:
(i) polymerization of the polymer P1 from a first mixture of monomers, distinct from each other, comprising:
- at least one anionic monomer (a) having a polymerizable vinyl group,
- at least one nonionic hydrophobic monomer (b) having a polymerizable vinyl group,
- at least one cross-linking monomer (c),
- at least one associative monomer (d) having a polymerizable vinyl group and a hydrophobic hydrocarbon chain and
- optionally at least one additional nonionic monomer (e), distinct from the monomer (b) and
(ii) polymerization, in the presence of the polymer P1 obtained above, of a second mixture of monomers, distinct from each other, comprising:
- at least one anionic monomer (a') having a polymerizable vinyl group,
- at least one nonionic hydrophobic monomer (b') having a polymerizable vinyl group,
- at least one cross-linking monomer (c'),
- optionally an associative monomer (d') having a polymerizable vinyl group and a hydrophobic hydrocarbon chain and
- optionally at least one additional nonionic monomer (e'), distinct from the monomer (b').

12. An aqueous composition comprising at least one multiphasic polymer as defined according to any one of claims 1 to 10 or as obtained according to the method of claim 11.

13. The composition according to the preceding claim, said composition having a pH value of less than or equal to 6, in particular of less than or equal to 5.5 and more particularly of less than or equal to 5.

14. The composition according to claim 12 or 13, comprising from 0.1% to 20% by weight, in particular from 0.5% to 12% by weight of multiphasic polymer(s) based on its total weight.

15. A use of a multiphasic polymer as defined according to any one of claims 1 to 10 or as obtained according to the method of claim 11, as thickening and suspending agent.
